# EUROPEAN PATENT APPLICATION

(11) **EP 4 268 827 A1**
(43) Date of publication of application: **01.11.2023**
(21) Application number: 21910965.9
(22) Date of filing: 23.12.2021
(51) Int. Cl.: A61K 31/551, A61K 9/06, A61K 9/107, A61K 47/06, A61K 47/10, A61K 47/12, A61K 47/14, A61K 47/44, A61P 27/02, A61P 27/14, A61P 37/08

(54) **PHARMACEUTICAL COMPOSITION FOR TOPICAL ADMINISTRATION CONTAINING EPINASTINE OR SALT THEREOF**

(30) Priority: 24.12.2020 JP 2020215231; 09.09.2021 JP 2021146903
(71) Applicant: Santen Pharmaceutical Co., Ltd., Kita-ku Osaka-shi Osaka 530-8552 (JP)
(72) Inventor: OGURA, Naoki, Ikoma-shi, Nara 630-0101 (JP); KAJIWARA, Yu, Osaka-shi, Osaka 530-8552 (JP); FUJISAWA, Koushi, Ikoma-shi, Nara 630-0101 (JP); ESAKI, Yoshihiko, Ikoma-shi, Nara 630-0101 (JP); SAKANAKA, Koji, Ikoma-shi, Nara 630-0101 (JP); FUJISAWA, Toyomi, Ikoma-shi, Nara 630-0101 (JP); TONE, Yuko, Osaka-shi, Osaka 530-8552 (JP)
(74) Representative: Müller-Boré & Partner Patentanwälte PartG mbB
(86) International application number: PCT/JP2021/047886
(87) International publication number: WO 2022/138826

(57) **Abstract**

The present invention provides a pharmaceutical composition for application to the skin comprising epinastine or a salt thereof in a concentration of 0.05 to 1% (w/w) as an active ingredient which can maintain the concentration of the active ingredient for a long time even at lower concentrations.

## Description

### TECHNICAL FIELD

The present invention relates to a pharmaceutical composition for application to the skin comprising epinastine or a salt thereof (hereinafter also referred to as "the pharmaceutical composition of the present invention").

### BACKGROUND ART

For example, Alesion^{®} LX ophthalmic solution 0.1% comprising epinastine hydrochloride as an active ingredient, which is used as an agent for treating allergic conjunctivitis, is currently marketed as pharmaceutical products comprising epinastine or a salt thereof. Such ophthalmic solution is usually used in the dose and usage of 1 drop per time, twice daily (Non-Patent Document 1). In an ophthalmic solution, it is desirable to reduce the administration frequency thereof in terms of medication adherence. On the other hand, the reduced administration frequency thereof may reduce the drug efficacy of an active ingredient because the effective concentration of the active ingredient in an ocular tissue is not maintained. Hence, it is required to maintain the effective concentration thereof in an ocular tissue to produce the drug efficacy. As a method of maintaining the effective concentration of an active ingredient in an ocular tissue, there is a method of increasing the concentration of an active ingredient in formulation. On the other hand, an increase in the concentration of the active ingredient in formulation may enhance the risk of developing side effects. Actually, Non-Patent Document 1 suggests that the formulation causes eye irritation as the concentration of epinastine hydrochloride in formulation increases. In view of Non-Patent Document 1, it is thought that there is an upper limit for the concentration of an active ingredient in an ophthalmic solution which can be safely administered.

In order to maintain the effective concentration of an active ingredient in an ocular tissue, the attempts to select an administration method other than ophthalmic administration have recently been done. For example, Patent Document 1 discloses a transdermal absorption formulation (particularly, a patch formulation) for treatment of an ophthalmic disease having a structure that a plaster layer containing an agent for treating an ophthalmic disease is provided on a support for adhering the transdermal absorption formulation to a skin surface including a front surface of an eyelid to percutaneously transfer the agent for treating the ophthalmic disease in the plaster layer to an ophthalmic topical tissue by percutaneous permeation substantially without being administered through a systemic blood flow. Also, an ophthalmic transdermal absorption formulation has been reported as a formulation comprising epinastine or a salt thereof other than an ophthalmic solution (Patent Document 2). Patent Document 2 reports that the group attached with the patch formulation comprising epinastine hydrochloride in a high concentration of 10% (w/w) shows the anti-allergic effect for a longer time than the group administrated with the ophthalmic solution comprising epinastine hydrochloride in a concentration of 0.05% (w/v).

### PRIOR ART DOCUMENTS

### PATENT DOCUMENTS

Patent Document 1: WO 2004/064817
Patent Document 2: WO 2007/007851

### NON-PATENT DOCUMENTS

Non-Patent Document 1: Alesion^{®} LX ophthalmic solution 0.1%, pharmaceutical interview form

### SUMMARY OF INVENTION

### (PROBLEM TO BE SOLVED BY THE INVENTION)

A transdermal absorption formulation is broadly classified into a patch formulation, a formulation for application to the skin, an aerosol formulation, and others. Among them, the patch formulation is suitable for the sustained delivery of an active ingredient to an affected area on the skin surface or a local affected area through the skin surface because the patch formulation is used by applying a base comprising the active ingredient onto the skin surface over a long time. On the other hand, the patch formulation may cause symptoms such as dermatitis (rash) because it is adhered to the skin for a long time. The problem may be more likely to occur when adhered the patch formulation to the skin, particularly the thin and irritation-sensitive eyelid skin. Also, Patent Document 1 discloses that an ophthalmic ointment, which is a type of formulations for application to the skin, is better in the sustainability of drug efficacy than an ophthalmic solution, but it is difficult to exactly control the dose of the active ingredient in the ointment and also that the ophthalmic ointment may cause the reduction in visual acuity upon its application. That is, it is suggested that the ophthalmic ointment is a formulation with a high risk of occurring side effects in the treatment of ophthalmic diseases. In addition, an ointment formulation, which is a type of formulations for application to the skin, is not sufficient in terms of the usability of formulation because the ointment formulation consisting only of oleaginous bases is sticky, difficult to wash off with water and hard to spread it on the skin. A cream formulation, which is a type of formulations for application to the skin, is superior to the ointment in the terms of the usability of formulation because the cream formulation is smoother and easy to wash off with water and to spread it on the skin, but the cream formulation comprising a hydrophilic substance carries the risk of causing the skin irritation due to various additives such as preservative and surfactant.

In light of the above, it is desirable to reduce the concentration of the active ingredient added in a formulation in terms of side effects and to reduce the administration frequency thereof in terms of medication adherence in developing a transdermal absorption formulation, for example, a formulation for application to the skin. However, an agent for treating allergic conjunctivitis for application to the skin that has low skin irritation as well as is effective at low concentrations and once-daily dosing has not been known yet and has not been marketed as a pharmaceutical product.

Hence, an object of the present invention is to provide an agent for treating allergic conjunctivitis for application to the skin that is effective at low concentrations and once-daily dosing, particularly a novel pharmaceutical composition comprising epinastine or a salt thereof as an active ingredient.

### (MEANS FOR SOLVING THE PROBLEMS)

The present inventors have intensively studied to develop a novel pharmaceutical composition comprising epinastine or a salt thereof as an active ingredient, and then have found that when a pharmaceutical composition comprising epinastine or a salt thereof in low concentrations is administered to the eyelid skin, the active ingredient retained in the eyelid skin tissue is gradually transferred to an ocular tissue, and thus the concentration of the active ingredient in the ocular tissue is maintained for a long time to produce the therapeutic effect as an agent for treating allergic conjunctivitis. In addition, the present inventors have found that the pharmaceutical composition comprising epinastine or a salt thereof at low concentrations has low skin irritation, and thus can minimize safety concerns even when administered to the thin and irritation-sensitive eyelid skin and produce good usability of formulation by the adjustment of the viscosity thereof. Based upon the new findings, the present invention has been completed.

Specifically, the present invention provides the following embodiments.
(1) A pharmaceutical composition for application to the skin comprising epinastine or a salt thereof in a concentration of 0.05 to 1% (w/w) as an active ingredient.
(2) The pharmaceutical composition according to the above item (1) for use in the administration to the eyelid skin.
(3) The pharmaceutical composition according to the above item (1) or (2) for use in the administration to a patient once daily.
(4) The pharmaceutical composition according to any one of the above items (1) to (3) for treating allergic conjunctivitis.
(5) The pharmaceutical composition according to any one of the above items (1) to (4) with a viscosity of 150 Pa·s or less at 20 degrees Celsius.
(6) The pharmaceutical composition according to any one of the above items (1) to (5), wherein the pharmaceutical composition is ointment formulation, cream formulation or gel formulation.
(7) The pharmaceutical composition according to any one of the above items (1) to (5), wherein the pharmaceutical composition is cream formulation.
(8) The pharmaceutical composition according to any one of the above items (1) to (7), wherein the pharmaceutical composition is water-in-oil emulsion.
(9) The pharmaceutical composition according to any one of the above items (1) to (8), wherein the concentration of the epinastine or a salt thereof is 0.05% (w/w).
(10) The pharmaceutical composition according to any one of the above items (1) to (8), wherein the concentration of the epinastine or a salt thereof is 0.5% (w/w).
(11) The pharmaceutical composition according to any one of the above items (1) to (10), wherein the epinastine or a salt thereof is epinastine hydrochloride.
(12) The pharmaceutical composition according to any one of the above items (1) to (10), wherein the epinastine or a salt thereof is epinastine.
(13) The pharmaceutical composition according to any one of the above items (1) to (12) which comprises one or more oil ingredients selected from the group consisting of a hydrocarbon, a wax, an oil and fat, an aliphatic carboxylic acid or a salt thereof, a fatty acid ester and a higher alcohol.
(14) The pharmaceutical composition according to any one of the above items (1) to (12) which comprises a surfactant.
(15) The pharmaceutical composition according to the above item (14), wherein the surfactant is glycerin fatty acid ester.
(16) The pharmaceutical composition according to the above item (14) or (15), wherein the surfactant has a HLB of 3.0 to 6.0.
(17) The pharmaceutical composition according to any one of the above items (1) to (16), wherein the pharmaceutical composition is free of a paraben.
(18) A pharmaceutical composition for application to the skin comprising epinastine or a salt thereof in a concentration of 0.05 to 0.5% (w/w) as an active ingredient, wherein the pharmaceutical composition is cream formulation in the form of water-in-oil emulsion for use in the administration to the eyelid skin once daily.
(19) A pharmaceutical composition for application to the skin comprising epinastine or a salt thereof in a concentration of 0.5% (w/w) as an active ingredient, wherein the pharmaceutical composition is cream formulation in the form of water-in-oil emulsion for use in the administration to the eyelid skin once daily.
(20) A method of transferring a therapeutically effective amount of epinastine to an ocular tissue, by administering a pharmaceutical composition comprising epinastine or a salt thereof in a concentration of 0.05 to 1% (w/w) as an active ingredient to the eyelid skin.
(21) A method of producing the sustained release of a therapeutically effective amount of epinastine from eyelid skin to an ocular tissue, by administering a pharmaceutical composition comprising epinastine or a salt thereof in a concentration of 0.05 to 1% (w/w) as an active ingredient to the eyelid skin.
(22) The method according to the above item (20) or (21), wherein the pharmaceutical composition is administered to a patient once daily.
(23) The method according to any one of the above items (20) to (22), wherein the ocular tissue is conjunctiva.

In addition, the present invention provides the following embodiments.
(24) A method of treating allergic conjunctivitis, which comprises administering a therapeutically effective amount of the pharmaceutical composition according to any one of the above items (1) to (19) in a patient in need thereof.
(25) Use of the pharmaceutical composition according to any one of the above items (1) to (19) in the manufacture of a medicament for treating allergic conjunctivitis.

Each feature of the above (1) to (25) may be optionally selected and combined two or more.

### (EFFECTS OF THE INVENTION)

According to the present invention, a pharmaceutical composition for application to the skin comprising epinastine or a salt thereof as an active ingredient that produces sufficient therapeutic effects even at low concentrations and reduced administration frequency thereof can be provided.

The present invention can use epinastine at low concentrations, and thus can sufficiently ensure the safety as pharmaceutical products. In addition, the present invention has low skin irritation, and thus can minimize safety concerns even when administered to an irritation-sensitive tissue (e.g., eyelid skin).

The present invention can improve the usability of pharmaceutical composition by adjusting the viscosity of pharmaceutical composition, for example, adjusting the viscosity thereof at 20 degrees Celsius to 150 Pa·s or less.

### DETAILED DESCRIPTION OF THE PREFERRED EMBODIMENTS

Hereinafter, the present invention is explained in detail.

"Epinastine" as used herein is a compound represented by chemical name: (±)-3-Amino-9,13b-dihydro-1H-dibenz[c,f]imidazo[1,5-a]azepine, and also the following formula:

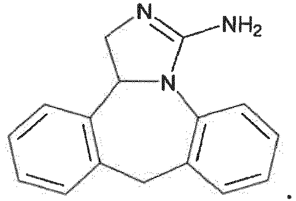

Epinastine in the pharmaceutical composition of the present invention may be in the form of racemate or optical isomer.

Epinastine in the pharmaceutical composition of the present invention may be in a salt form, and it is not particularly limited as long as the salt is a pharmaceutically acceptable salt. Examples thereof include salts with an inorganic acid, an organic acid, and others.

Examples of the salts with an inorganic acid include salts with hydrochloric acid, hydrobromic acid, hydroiodic acid, nitric acid, sulfuric acid, phosphoric acid, and others.

Examples of the salts with an organic acid include salts with acetic acid, oxalic acid, fumaric acid, maleic acid, succinic acid, malic acid, citric acid, tartaric acid, adipic acid, gluconic acid, glucoheptonic acid, glucuronic acid, terephthalic acid, methanesulfonic acid, alanine, lactic acid, hippuric acid, 1,2-ethanedisulfonic acid, isethionic acid, lactobionic acid, oleic acid, gallic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid, sulfosalicylic acid, and others.

Particularly preferably, the salt of epinastine is monohydrochloride (epinastine hydrochloride).

Epinastine or a salt thereof in the pharmaceutical composition of the present invention may be in the form of a hydrate or a solvate.

When the pharmaceutical composition of the present invention is prepared, epinastine in the pharmaceutical composition may be in a salt form, but is more preferably in free form to enhance the transdermal absorption of epinastine more efficiently. In the preparation process of the pharmaceutical composition of the present invention, epinastine in free form may be used to prepare the pharmaceutical composition. Also, epinastine in free form may be generated in the pharmaceutical composition by the desalination of a salt of epinastine (e.g., epinastine hydrochloride) by the reaction of the salt of epinastine and an appropriate amount of a base (e.g., sodium hydroxide) in the preparation process.

Epinastine or a salt thereof in the pharmaceutical composition of the present invention may be contained in sufficient amounts to produce the desired therapeutic effect as a medicament, but it is difficult to produce the desired effect if the amount is too low. On the other hand, if the amount is too high, for example, when the pharmaceutical composition is administered to the skin, the risk of developing unexpected side effects is enhanced by retaining a large amount of the active ingredient in the skin tissue at the administration site, and it is not easy to remove the active ingredient retained in the skin tissue when side effects are developed. The lower limit for the amount of epinastine or a salt thereof is, for example, 0.01% (w/w), preferably 0.03% (w/w), more preferably 0.05% (w/w). The upper limit thereof is, for example, 3% (w/w), preferably 2% (w/w), more preferably 1% (w/w) or less than 1% (w/w). Also, the amount of epinastine or a salt thereof in the pharmaceutical composition of the present invention is preferably 0.03 to 2% (w/w), more preferably 0.05 to 1% (w/w) or less than 0.05 to 1% (w/w), furthermore preferably 0.05 to 0.5% (w/w), particularly preferably 0.1 to 0.5% (w/w). Specifically, the amount of epinastine or a salt thereof is preferably 0.05% (w/w), 0.1% (w/w), 0.2% (w/w), 0.25% (w/w), 0.3% (w/w), 0.4% (w/w), 0.5% (w/w), 0.6% (w/w), 0.7% (w/w), 0.75% (w/w), 0.8% (w/w), 0.9% (w/w) or 1% (w/w) and particularly preferably 0.5% (w/w).

The term "% (w/w)" as used herein means the mass (g) of an object ingredient in 100 g of the pharmaceutical composition of the present invention. When the pharmaceutical composition of the present invention comprises a salt of epinastine, the value means the amount of the salt of epinastine. Also, when the pharmaceutical composition of the present invention comprises epinastine or a salt thereof in the form of a hydrate or a solvate, the value means the amount of the hydrate or the solvate of epinastine or a salt thereof. Hereinafter, the same shall apply to the followings unless otherwise specified.

The pharmaceutical composition of the present invention is preferably used in transdermal administration by application (application to the skin), but may be used in parenteral (e.g., topical) administration such as transdermal administration other than application to the skin.

The pharmaceutical composition of the present invention may be prepared as an external preparation. Also, it may be prepared as an external preparation for transdermal administration. The pharmaceutical composition of the present invention is preferably prepared as an external preparation for application to the skin. In addition, the pharmaceutical composition of the present invention is used as preferably an ophthalmic external preparation, more preferably an ophthalmic transdermal absorption formulation, furthermore preferably an ophthalmic formulation for application to the skin.

The dosage form of the pharmaceutical composition of the present invention is not particularly limited as long as it is can be used as a pharmaceutical product. Examples thereof include ointment formulation, cream formulation, gel formulation, external solution (e.g., lotion formulation, liniment formulation), external solid formulation (e.g., external powder), and others. The dosage form thereof is preferably ointment formulation, cream formulation, gel formulation and external solution, more preferably ointment formulation, cream formulation and gel formulation, particularly preferably cream formulation.

They can be prepared according to a method commonly used in the art.

The pharmaceutical composition of the present invention is preferably administered to the area near the eye. The term "area near the eye" refers to the eyelids (e.g., the upper eyelid, the lower eyelid) and the area near the eyelid or the periorbital area. Also, the term includes the skin of each eyelid and the skin of the area near the eyelid or the periorbital area skin. The administration to the area near the eye includes, for example, the application to the skin of the upper eyelid, the lower eyelid or both eyelids and the area near the eyelid or the application to the periorbital area skin. The edge of eyelid may be included in the area near the eye. On the other hand, when a portion of the pharmaceutical composition administered to the edge of eyelid comes in contact with the sensitive ocular surface with a blink of eyes, the possibility of producing ocular irritation is increased. Hence, it is more preferable not to administer the pharmaceutical composition to the edge of eyelid, and it is furthermore preferable not to administer the pharmaceutical composition intraocularly.

When the pharmaceutical composition of the present invention is administered to the area near the eye, the strong pressure to the eyelid skin may cause pain to the eyeball located directly under the eyelid skin and affect its vision because the eyelid skin is a very thin and soft tissue. Hence, when the pharmaceutical composition of the present invention is administered to the area near the eye, it is preferable not to spread and apply it on the eyelid skin while adding strong pressure but to easily and uniformly apply it on the eyelid skin so that it is smoothly stretched. In addition, when a drug solution is in the liquid form such as water, it can be easily applied to eyelid skin without pressure, but may cause irritation or discomfort feelings when the drug solution drips and the dripping solution gets into the eye(s) or mouth. Hence, it is preferable that the pharmaceutical composition of the present invention is viscous to the extent that it does not drip when administered to the eyelid skin.

In the pharmaceutical composition of the present invention, the viscosity at 20 degrees Celsius is, for example, 300 Pa·s (Pascal·second) or less, preferably 200 Pa·s or less, more preferably 150 Pa·s or less, furthermore preferably 100 Pa·s or less, particularly preferably 80 Pa· s or less. Also, the viscosity is, for example, 0.001 Pa·s or more, preferably 0.01 Pa·s or more, more preferably 0.1 Pa·s or more, furthermore preferably 1 Pa·s or more, particularly preferably 10 Pa·s or more. Also, the viscosity is, for example, 0.001 to 300 Pa·s, preferably 0.01 to 200 Pa·s, more preferably 0.1 to 150 Pa·s, furthermore preferably 1 to 100 Pa·s, particularly preferably 10 to 80 Pa·s. Also, the viscosity may be more preferably 5 to 150 Pa·s, 5 to 100 Pa·s, 10 to 150 Pa·s or 10 to 100 Pa·s.

The viscosity of the pharmaceutical composition of the present invention may be measured according to, for example, the viscosity measurement method described in the General Test Method of the Japanese Pharmacopoeia 17th Edition.

The pharmaceutical composition of the present invention may comprise an additive for pharmaceutical products as appropriate. For example, an additive such as pH adjuster, buffering agent, tonicity agent, viscosity agent, stabilizing agent, antioxidant, preservative, surfactant, cooling agent and oil ingredient may be added. They may be used alone, respectively, and two or more of them may be used in combination. Also, they may be added in an appropriate amount.

In the pharmaceutical composition of the present invention, a pH adjuster available as pharmaceutical additives may be added at appropriate. Examples of the pH adjuster include an acid or a base. Examples of the acid include hydrochloric acid, phosphoric acid, acetic acid, citric acid, a salt thereof, and others. Examples of the base include sodium hydroxide, potassium hydroxide, sodium carbonate, sodium hydrogen carbonate, and others. They may be in form of a hydrate or a solvate. When the pH adjuster is a base, the base may be added to generate epinastine in the free form by the desalination of a salt of epinastine.

The pH of the pharmaceutical composition of the present invention is not limited as long as it is within a range acceptable for pharmaceutical products. The pH thereof is within a range of, for example, 4.0 to 8.5 or 4.0 to 8.0, preferably 6.0 to 8.0, more preferably 6.5 to 7.5. The pH thereof is particularly preferably 6.7 to 7.3. Also, the pH thereof may be 6.7, 6.8, 6.9, 7.0, 7.1, 7.2 or 7.3.

In the pharmaceutical composition of the present invention, a buffering agent available as pharmaceutical additives may be added at appropriate. Examples of the buffering agent include phosphoric acid or a salt thereof, boric acid, borax, trometamol, an organic acid or a salt thereof, and others. They may be in the form of a hydrate or a solvate.

Examples of the phosphoric acid or a salt thereof include phosphoric acid, sodium phosphate, sodium dihydrogen phosphate, disodium hydrogen phosphate, potassium phosphate, potassium dihydrogen phosphate, dipotassium hydrogen phosphate, and others.

Examples of the organic acid include citric acid, acetic acid, ε-aminocaproic acid, gluconic acid, fumaric acid, lactic acid, ascorbic acid, succinic acid, maleic acid, malic acid, amino acid, and others. Examples of the salt thereof include sodium salt, potassium salt, and others.

The amount of the buffering agent in the pharmaceutical composition of the present invention may be suitably varied depending on, for example, the type of the buffering agent. The amount thereof is, for example, 0.01 to 5% (w/w), preferably 0.05 to 3% (w/w), more preferably 0.1 to 2% (w/w), furthermore preferably 0.1 to 1% (w/w), but this is not applied if the buffering agent produces any effect other than the effect as buffering agent. Also, when the pharmaceutical composition of the present invention comprises a buffering agent, the buffering agent may be used alone and two or more buffering agents may be used in combination.

Phosphoric acid or a salt thereof and the organic acid or a salt thereof in the pharmaceutical composition of the present invention may act as pH adjuster and buffering agent.

In the pharmaceutical composition of the present invention, a tonicity agent available as pharmaceutical additives may be added as appropriate. Examples of the tonicity agent include an ionic tonicity agent, a non-ionic tonicity agent, and others. The tonicity is preferably an ionic tonicity agent.

Examples of the ionic tonicity agent include sodium chloride, potassium chloride, calcium chloride, magnesium chloride, and others. They may be in the form of a hydrate or a solvate.

Examples of the non-ionic tonicity agent include glycerin (concentrated glycerin), propylene glycol, 1,3-butylene glycol, polyethylene glycol, sorbitol, mannitol, trehalose, maltose, sucrose, xylitol, and others. They may be in the form of a hydrate or a solvate.

The amount of the tonicity agent in the pharmaceutical composition of the present invention may be suitably varied depending on, for example, the type of the tonicity agent. The amount thereof is, for example, 0.01 to 5% (w/w), preferably 0.05 to 3% (w/w), more preferably 0.1 to 2% (w/w), furthermore preferably 0.1 to 1% (w/w), but this is not applied if the tonicity agent produces any effect other than the effect as tonicity agent. Also, when the pharmaceutical composition of the present invention comprises a tonicity agent, the tonicity agent may be used alone and two or more tonicity agents may be used in combination.

In the pharmaceutical composition of the present invention, a viscosity agent available as pharmaceutical additives may be added as appropriate. Examples of the viscosity agent include a cellulosic polymer, polyvinylpyrrolidone, a carboxyvinyl polymer, a mucopolysaccharide and a polyhydric alcohol. They may be in the form of a salt, a hydrate or a solvate. Examples of the cellulosic polymer include methyl cellulose, ethyl cellulose, hydroxymethylcellulose, hydroxyethyl cellulose, hydroxypropyl cellulose, hydroxyethyl methyl cellulose, hydroxypropyl methyl cellulose, carboxymethyl cellulose, sodium carboxymethyl cellulose, hydroxypropyl methylcellulose acetate succinate, hydroxypropylmethyl cellulose phthalate, carboxymethylethyl cellulose, cellulose acetate phthalate, and others. Examples of the carboxyvinyl polymer include carbopol, and others. Examples of the polyhydric alcohol include polyvinyl alcohol, polyethylene glycol, and others. Examples of the mucopolysaccharide include hyaluronic acid, sodium hyaluronate, chondroitin sulfate, and others. The viscosity agent is preferably a cellulosic polymer.

The amount of the viscosity agent in the pharmaceutical composition of the present invention may be suitably varied depending on, for example, the type of the viscosity agent. The amount thereof is, for example, 0.01 to 20% (w/w), preferably 0.1 to 10% (w/w), more preferably 1 to 10% (w/w), but this is not applied if the viscosity agent produces any effect other than the effect as viscosity agent. Also, when the dosage form of the pharmaceutical composition of the present invention is gel formulation, the amount thereof may be, for example, 20% (w/w) or more. In addition, when the pharmaceutical composition of the present invention comprises a viscosity agent, the viscosity agent may be used alone and two or more viscosity agents may be used in combination.

In the pharmaceutical composition of the present invention, a stabilizing agent available as pharmaceutical additives may be added at appropriate. Examples of the stabilizing agent include edetic acid or a salt, cyclodextrin, and others. They may be in the form of a hydrate or a solvate. The stabilizing agent is preferably edetic acid or a salt thereof.

Examples of the edetic acid or a salt thereof include edetic acid, disodium edetate (sodium edetate), tetrasodium edetate, and others.

The amount of the stabilizing agent in the pharmaceutical composition of the present invention may be suitably varied depending on, for example, the type of the stabilizing agent. The amount thereof is, for example, 0.01 to 5% (w/w), preferably 0.01 to 3% (w/w), more preferably 0.01 to 1% (w/w), but this is not applied if the stabilizing agent produces any effect other than the effect as stabilizing agent. In addition, when the pharmaceutical composition of the present invention comprises a stabilizing agent, the stabilizing agent may be used alone and two or more stabilizing agents may be used in combination.

In the pharmaceutical composition of the present invention, an antioxidant available as pharmaceutical additives may be added as appropriate. Examples of the antioxidant include ascorbic acid, sodium ascorbate, tocopherol, tocopherol acetate, citric acid, sodium citrate, potassium citrate, dibutylhydroxytoluene, propyl gallate, cysteine, N-acetylcysteine, methionine, sodium bisulfite, sodium sulfite, sodium thiosulfate, benzotriazole, 2-mercaptobenzimidazole, and others. They may be in the form of a hydrate or a solvate. Also, when the antioxidant has one or more chiral centers, it may be used as racemate or optical isomer. The antioxidant is preferably dibutylhydroxytoluene, 2-mercaptobenzimidazole.

The amount of the antioxidant in the pharmaceutical composition of the present invention may be suitably varied depending on, for example, the type of the antioxidant. The amount thereof is, for example, 0.01 to 2% (w/w), preferably 0.01 to 1% (w/w), more preferably 0.01 to 0.5% (w/w), but this is not applied if the antioxidant produces any effect other than the effect as antioxidant. In addition, when the pharmaceutical composition of the present invention comprises an antioxidant, the antioxidant may be used alone and two or more antioxidants may be used in combination.

In the pharmaceutical composition of the present invention, a preservative available as pharmaceutical additives may be added at appropriate. Examples of the preservative include benzalkonium chloride, benzalkonium bromide, benzethonium chloride, chlorhexidine gluconate, chlorhexidine hydrochloride, a paraben, sodium chlorite, phenoxyethanol, thymol, sorbic acid, chlorobutanol, and others.

Examples of the paraben include methyl paraoxybenzoate, ethyl paraoxybenzoate, propyl paraoxybenzoate, and others.

The amount of the preservative in the pharmaceutical composition of the present invention may be suitably varied depending on, for example, the type of the preservative. The amount thereof is, for example, 0.001 to 1% (w/w), but this is not applied if the preservative produces any effect other than the effect as preservative. In addition, when the pharmaceutical composition of the present invention comprises a preservative, the preservative may be used alone and two or more preservatives may be used in combination.

When the pharmaceutical composition of the present invention can produce the preservative efficacy required for pharmaceutical products without adding a preservative therein, it is preferable not to add a preservative, for example, a paraben in the pharmaceutical composition. For example, the criteria for evaluating the preservative efficacy required for pharmaceutical products is defined for each formulation category in the Japanese Pharmacopoeia 17th Edition. When each formulation meets the criteria, it is evaluated that the formulation has the preservative efficacy. Also, since epinastine or a salt thereof produces the preservative efficacy depending on the concentration thereof, the pharmaceutical composition of the present invention can achieve the desired preservative efficacy without the addition of a paraben, depending on the type of formulation. The desired preservative efficacy means, for example, that a formulation meets the criteria defined in the preservatives-effectiveness tests described in the Japanese Pharmacopoeia 17th Edition. Also, the irritation when the pharmaceutical composition of the present invention is applied to the skin can be reduced because no preservative is contained therein.

In the pharmaceutical composition of the present invention, a surfactant available as pharmaceutical additives may be added as appropriate. Examples of the surfactant include a cationic surfactant, an anionic surfactant, a non-ionic surfactant, and others.

Examples of the cationic surfactant include alkylamine salt, alkylamine-polyoxyethylene additive, fatty acid triethanolamine monoester salt, acylaminoethyldiethylamine salt, fatty acid polyamine conjugate, alkyl imidazoline, 1-acylaminoethyl-2-alkylimidazoline, 1-hydroxylethyl-2-alkylimidazoline, and others.

Examples of the anionic surfactant include sodium alkylbenzene sulfonate, sodium dodecyl sulfate, a phospholipid such as lecithin, and others.

Examples of the non-ionic surfactant include polyoxyethylene fatty acid ester, sorbitan fatty acid ester, polyoxyethylene sorbitan fatty acid ester, polyoxyethylene hydrogenated castor oil, polyoxyl castor oil, polyoxyethylene polyoxypropylene glycol, poloxyethylene polyoxypropylene cetyl ether, polyoxyethylene alkyl ether, glycerin fatty acid ester, sucrose fatty acid ester, and others.

Examples of the polyoxyethylene fatty acid ester include polyoxyl 40 stearate, polyoxyl 45 stearate, polyoxyl 55 strearate, polyethylene glycol myristate, polyethylene glycol monooleate, polyethylene glycol monostearate, polyethylene glycol monoisostearate, polyethylene glycol monolaurate, and others.

Examples of the sorbitan fatty acid ester include sorbitan caprylate, sorbitan laurylate, sorbitan stearate, sorbitan isostearate, sorbitan tristearate, sorbitan behenate, sorbitan tribehenate, sorbitan oleate, sorbitan trioleate, and others.

Examples of the polyoxyethylene sorbitan fatty acid ester include polysorbate 80, polysorbate 60, polysorbate 40, polysorbate 20, polyoxyethylene sorbitan monolaurate, polyoxyethylene sorbitan trioleate, polysorbate 65, and others.

Examples of the polyoxyethylene hydrogenated castor oil include polyoxyethylene hydrogenated castor oil 5, polyoxyethylene hydrogenated castor oil 10, polyoxyethylene hydrogenated castor oil 20, polyoxyethylene hydrogenated castor oil 40, polyoxyethylene hydrogenated castor oil 50, polyoxyethylene hydrogenated castor oil 60, polyoxyethylene hydrogenated castor oil 100, and others.

Examples of the polyoxyl castor oil include polyoxyl 5 castor oil, polyoxyl 9 castor oil, polyoxyl 15 castor oil, polyoxyl 35 castor oil, polyoxyl 40 castor oil, and others.

Examples of the polyoxyethylene polyoxypropylene glycol include polyoxyethylene (3) polyoxypropylene (17) glycol, polyoxyethylene (20) polyoxypropylene (20), polyoxyethylene (42) polyoxypropylene (67) glycol, polyoxyethylene (54) polyoxypropylene (39) glycol, polyoxyethylene (105) polyoxypropylene (5) glycol, polyoxyethylene (120) polyoxypropylene (40) glycol, polyoxyethylene (160) polyoxypropylene (30) glycol, polyoxyethylene (196) polyoxypropylene (67) glycol, and others.

Examples of the polyoxyethylene polyoxypropylene cetyl ether include polyoxyethylene (20) polyoxypropylene (4) cetyl ether, polyoxyethylene (20) polyoxypropylene (8) cetyl ether, and others.

Examples of the polyoxyethylene alkyl ether include polyoxyethylene cetyl ether, polyoxyethylene lauryl ether, polyoxyethylene behenyl ether, polyoxyethylene arachyl ether, and others.

Examples of the glycerin fatty acid ester include glyceryl monocaprylate, glyceryl dicaprylate, glyceryl tricaprylate, polyglyceryl monocaprylate, polyglyceryl dicaprylate, polyglyceryl tricaprylate, glyceryl monocaprate, glyceryl dicaprate, glyceryl tricaprate, polyglyceryl monocaprate, polyglyceryl dicaprate, polyglyceryl tricaprate, glyceryl monolaurate, glyceryl dilaurate, glyceryl trilaurate, polyglyceryl monolaurate, polyglyceryl dilaurate, polyglyceryl trilaurate, glyceryl monomyristate, glyceryl dimyristate, glyceryl trimyristate, polyglyceryl monomyristate, polyglyceryl dimyristate, polyglyceryl trimyristate, glyceryl monopalmitate, glyceryl dipalmitate, glyceryl tripalmitate, polyglyceryl monopalmitate, polyglyceryl dipalmitate, polyglyceryl tripalmitate, glyceryl monostearate, glyceryl distearate, glyceryl tristearate, polyglyceryl monostearate, polyglyceryl distearate, polyglyceryl tristearate, glyceryl monoisostearate, glyceryl diisostearate, glyceryl triisostearate, polyglyceryl monoisostearate, polyglyceryl diisostearate, polyglyceryl triisostearate, glyceryl monobehenate, glyceryl dibehenate, glyceryl tribehenate, polyglyceryl monobehenate, polyglyceryl dibehenate, polyglyceryl tribehenate, glyceryl monooleate, glyceryl dioleate, glyceryl trioleate, polyglyceryl monooleate, polyglyceryl dioleate, polyglyceryl trioleate, glyceryl monolinoleate, glyceryl dilinoleate, glyceryl trilinoleate, polyglyceryl monolinoleate, polyglyceryl dilinoleate, polyglyceryl trilinoleate, glyceryl monolinolenate, glyceryl dilinolenate, glyceryl trilinolenate, polyglyceryl monolinolenate, polyglyceryl dilinolenate, polyglyceryl trilinolenate, glyceryl monoricinoleate, glyceryl diricinoleate, glyceryl triricinoleate,, polyglyceryl monoricinoleate, polyglyceryl diricinoleate, polyglyceryl triricinoleate, condensed ricinoleic acid polyglyceryl ester (also referred to as polyglyceryl polyricinoleate, condensed ricinolenic acid polyglyceryl ester or polyglyceryl polyricinolenate), glyceryl monoarachidonate, glyceryl diarachidonate, glyceryl triarachidonate, polyglyceryl monoarachidonate, polyglyceryl diarachidonate, polyglyceryl triarachidonate, and others.

Examples of the sucrose fatty acid ester include sucrose stearate, sucrose palmitate, sucrose oleate and others.

The surfactant is more preferably a non-ionic surfactant, furthermore preferably glycerin fatty acid ester. For example, the surfactant is condensed ricinoleic acid polyglyceryl ester.

The amount of the surfactant in the pharmaceutical composition of the present invention may be suitably varied depending on, for example, the type of the surfactant. The amount thereof is, for example, 0.1 to 20% (w/w), preferably 0.5 to 10% (w/w), more preferably 1 to 5% (w/w), but this is not applied if the surfactant produces any effect other than the effect as surfactant. In addition, when the pharmaceutical composition of the present invention comprises a surfactant, the surfactant may be used alone and two or more surfactants may be used in combination. Also, the surfactant may be used as a mixture of two or more surfactants.

The surfactant is characterized by the balance between the hydrophilic and lipophilic portions of molecules thereof and has a hydrophile-lipophile balance (HLB) value. The HLB value increases with increasing the hydrophilicity of a molecule and may vary by the manufacturer even with the same ingredient name.

The HLB value of the surfactant in the pharmaceutical composition of the present invention is not particularly limited as long as it is a value that can be prepared as a formulation for application to the skin. The HLB value is, for example, 1 to 20, preferably 1.0 to 10.0, more preferably 2.0 to 8.0, furthermore preferably 3.0 to 6.0. Also, the surfactant is preferably a non-ionic surfactant of a HLB of 1.0 to 10.0, more preferably a non-ionic surfactant of a HLB of 2.0 to 8.0, furthermore preferably a non-ionic surfactant of a HLB of 3.0 to 6.0. Also, the surfactant is preferably glycerin fatty acid ester of a HLB of 1.0 to 10.0, more preferably glycerin fatty acid ester of a HLB of 2.0 to 8.0, furthermore preferably glycerin fatty acid ester of a HLB of 3.0 to 6.0. Examples of the glycerin fatty acid ester of a HLB of 3.0 to 6.0 include glyceryl monostearate, diglyceryl monostearate (polyglyceryl-2 stearate), tetraglyceryl monostearate (polyglyceryl-4 stearate), glyceryl monoisostearate, diglyceryl monoisostearate (polyglyceryl-2 isostearate), decaglyceryl pentastearate (polyglyceryl-10 pentastearate), decaglyceryl pentaisostearate (polyglyceryl-10 pentaisostearate), glyceryl myristate, polyglyceryl ricinoleate, condensed ricinoleic acid polyglyceryl ester, diglyceryl monooleate (polyglyceryl-2 oleate), tetraglyceryl monooleate (polyglyceryl-4 oleate), decaglyceryl pentaoleate (polyglyceryl-10 pentaoleate), and others.

In the pharmaceutical composition of the present invention, a cooling agent available as pharmaceutical additives may be added as appropriate. Examples of the cooling agent include terpenoid, essential oil containing terpenoid, and others.

Examples of the terpenoid include menthol, camphor, borneol, geraniol, nerol, cineole, citronellol, carvone, anethole, eugenol, limonene, linalool, linalyl acetate, and others. They may be in the form of d-, l- or dl-body.

Examples of the essential oil containing terpenoid include eucalyptus oil, bergamot oil, peppermint oil, fennel oil, rose oil, cassia oil, spearmint oil, camphor oil, cool mint, mentha oil, and others.

The amount of the cooling agent in the pharmaceutical composition of the present invention may be suitably varied depending on, for example, the type of the cooling agent. The amount thereof is, for example, 0.001 to 1% (w/w). In addition, when the pharmaceutical composition of the present invention comprises a cooling agent, the cooling agent may be used alone and two or more cooling agents may be used in combination.

In the pharmaceutical composition of the present invention, an oil ingredient available as pharmaceutical additives may be added at appropriate. Examples of the oil ingredient include a hydrocarbon, a wax, an oil and fat, an aliphatic carboxylic acid or a salt thereof, a fatty acid ester, medium-chain triglyceride (MCT), a higher alcohol, and others.

Examples of the hydrocarbon include vaseline, white petrolatum, liquid paraffin (light liquid paraffin, heavy liquid paraffin), solid paraffin (paraffin), squalene, squalane, ceresin, microcrystalline wax, and others. They may be used as a mixture thereof. Examples of the mixture include mixture of paraffin and microcrystalline wax, and others.

Examples of the wax include beeswax, white beeswax, lanolin, and others.

Examples of the oil and fat include olive oil, castor oil, sesame oil, soybean oil, and others.

Examples of the aliphatic carboxylic acid include short-chain fatty acid such as butyric acid, valeric acid and caproic acid, medium-chain fatty acid such as caprylic acid and capric acid, long-chain fatty acid such as lauric acid, myristic acid, palmitic acid, stearic acid, behenic acid, oleic acid, linoleic acid, linolenic acid, ricinoleic acid (ricinolenic acid) and arachidonic acid, aliphatic dicarboxylic acid such as succinic acid, glutaric acid, adipic acid and sebacic acid, and others. It also includes branched aliphatic carboxylic acid. Examples of the salt of the aliphatic carboxylic acid include sodium salt thereof, and others.

The fatty acid ester as used herein refers to a compound generated by the ester binding of the carboxyl group of an aliphatic carboxylic acid with an alcohol. Examples of the alcohol that may be esterified to the carboxyl group of an aliphatic carboxylic acid include a monovalent alcohol such as methanol, ethanol, n-propanol, n-butanol, n-pentanol, n-hexanol, n-heptanol, n-octanol, isopropanol, isobutanol, sec-butanol and isopentanol, a polyhydric alcohol such as ethylene glycol, diethylene glycol, propylene glycol, butyl alcohol and glycerin, a multimeric complex (polymer) of polyhydric alcohol such as dipolyethylene glycol, polyethylene glycol, dipropylene glycol, polypropylene glycol, diglycerin, triglycerin and polyglycerin, and others. Specific examples thereof include polyethylene glycol laurate, isopropyl myristate, octyl myristate, glycol palmitate, polyethylene stearate, isopropyl oleate, propylene glycol linoleate, ethyl linolenate, ethylene glycol ricinoleate (ricinolenate), diisopropyl adipate, and others. When the fatty acid ester has two or more ester bonds, the alcohols for forming the ester bonds may be the same or different.

Examples of the higher alcohol include lauryl alcohol, myristyl alcohol, palmityl alcohol (cetyl alcohol), stearyl alcohol, behenyl alcohol, oleyl alcohol, linoleyl alcohol, linolenyl alcohol, octydodecanol, and others.

When the oil ingredient in the pharmaceutical composition of the present invention acts as surfactant, the oil ingredient may be read as surfactant.

When the oil ingredient in the pharmaceutical composition of the present invention acts as a solubilizing agent (solubilizer), the oil ingredient may be read as solubilizing agent.

The amount of the oil ingredient in the pharmaceutical composition of the present invention may be suitably varied depending on, for example, the type of the oil ingredient. The amount thereof is, for example, 0.1 to 50% (w/w), preferably 1 to 40% (w/w), more preferably 10 to 30% (w/w). When the dosage form of the pharmaceutical composition of the present invention is ointment formulation, the amount thereof may be, for example, 50% (w/w) or more. In addition, when the pharmaceutical composition of the present invention comprises an oil ingredient, the oil ingredient may be used alone and two or more oil ingredients may be used in combination.

The pharmaceutical composition may further comprise a solvent and/or a dispersion medium. The pharmaceutical composition of the present invention comprising a solvent and/or a dispersion medium may be in the state that all of the ingredients contained therein are dissolved or a portion thereof is suspended, or may be in the form of emulsion or semi-solid. Examples of the solvent and/or the dispersion medium include water, ethanol, polyol (e.g., glycerin (glycerol), propylene glycol, 1,3-butylene glycol, liquid polyethylene glycol, macrogol), and others, but is not limited thereto.

The amount of a solvent and/or a dispersion medium in the pharmaceutical composition of the present invention may be suitably varied depending on, for example, the type of the solvent and/or the dispersion medium. For example, the amount thereof is preferably 10% (w/w) or more, more preferably 30% (w/w) or more, relative to the total amount of the pharmaceutical composition. In addition, when the pharmaceutical composition of the present invention comprises a solvent and/or a dispersion medium, the solvent and/or the dispersion medium may be used alone and two or more solvents and/or dispersion media may be used in combination.

When the pharmaceutical composition of the present invention is used as an emulsion, the pharmaceutical composition may be in the form of oil-in-water emulsion (emulsion consisting of oil droplets dispersed in water phase which is the continuous phase) or water-in-oil emulsion (emulsion consisting of aqueous droplets dispersed in oil phase which is the continuous phase). The pharmaceutical composition of the present invention is preferably water-in-oil emulsion.

The average size of the oil or aqueous droplets is, for example, 20 to 3000 nm, preferably 50 to 2000 nm, more preferably 100 to 1000 nm, furthermore preferably 200 to 800 nm.

The pharmaceutical composition of the present invention can be prepared according to a usual method commonly used in the art. For example, the pharmaceutical composition can be prepared by mixing an active ingredient with an additive such as a stabilizing agent, an antioxidant, a preservative, a surfactant, an oil ingredient as well as a solvent and/or a dispersion medium such as water. Also, the pharmaceutical composition can be prepared as a sterile formulation according to a usual sterilization method commonly used in the art as appropriate. The sterilization method is not particularly limited as long as it is a method which can be used in the preparation process. Examples thereof include highpressure steam sterilization, filtration sterilization, dry heat sterilization, electron beam (EB) sterilization, γ-ray sterilization, ethylene oxide gas (EOG) sterilization and hydrogen peroxide gas sterilization.

The pharmaceutical composition of the present invention may comprise a pharmaceutical active ingredient other than epinastine or a salt thereof, unless otherwise noted. Examples of other pharmaceutical active ingredient include an anti-inflammatory agent, an anti-bacterial agent, an anti-viral agent, a vitamin agent, a vasoconstrictive agent, a mydriatic agent, a miotic agent, an ocular hypotensive agent, an agent for treating dry eye, a local anesthetic agent, and others. Also, the pharmaceutical composition of the present invention may comprise epinastine or a salt thereof as the only active ingredient.

The pharmaceutical composition of the present invention is particularly useful as an agent for treating allergic conjunctivitis. The term "treatment of allergic conjunctivitis" as used herein refers to every treatment (e.g., improvement, alleviation, inhibition of progression) of allergic conjunctivitis or the associated symptoms and prophylaxis thereof.

The term "ocular tissue" as used herein includes, for example, conjunctiva, cornea, tear fluid, aqueous humor, anterior chamber. Particularly preferably, epinastine or a salt thereof is transferred into the conjunctiva to treat allergic conjunctivitis.

The term "patient" as used herein refers to human and an animal such as dog, cat, and house. Among them, the patient is preferably a mammal, more preferably human. The term "therapeutically effective amount" as used herein refers to an amount for producing the therapeutic effect on a disease or the associated symptoms or an amount for delaying the onset or progression of a disease or the associated symptoms, relative to untreated subjects.

The pharmaceutical composition of the present invention is preferably administered once daily, but the dose and usage thereof are not particularly limited as long as they are sufficient to produce the desired efficacy.

When the pharmaceutical composition of the present invention is used as ointment formulation, cream formulation or gel formulation, the dose thereof is not particularly limited as long as it is sufficient to produce the desired efficacy. Also, the dose varies depending on the amount of active ingredient and the patient. For example, a cream formulation may be applied to the skin at an appropriate amount, specifically about 1 mg to about 5 g, preferably about 5 mg to about 1 g, more preferably about 10 mg to about 500 mg, particularly preferably about 20 mg to about 100 mg, per time in an adult. For example, the dose thereof is 30 mg. As an example of the administration, a patient himself may take an appropriate amount, for example, about 20 to 40 mg of the pharmaceutical composition of the present invention prepared as an ophthalmic formulation for application to the skin on his finger and apply it to the upper and lower eyelid skin of one eye in about half the amount and then may apply it to the other eye in the same manner. In actual use, the patient may use the pharmaceutical composition in a rough amount without weighing it, using the aforementioned dose for each eye as a standard amount. In addition, the dose varies depending on the size and shape of the container filled with the pharmaceutical composition of the present invention, but the pharmaceutical composition may be used in an amount of 0.5 FTU or 1 FTU when the amount of the pharmaceutical composition loaded from the tip of an adult index finger to the first joint is defined as 1 FTU (1 Finger Tip Unit) as a standard amount.

The application time when applied to the skin is preferably 0.5 to 24 hours, more preferably 2 to 12 hours and furthermore preferably 4 to 8 hours. If the pharmaceutical composition is removed from the skin after the application time, a sufficient amount of the active ingredient therein is retained in the skin tissue. As a result, it is expected that the active ingredient is released slowly, resulting in the sustained efficacy. One example of the usage is to apply the pharmaceutical composition of the present invention to the eyelid skin before bedtime and remove it from the skin after waking up. Thereby, it is expected that the effect of treating and preventing allergic conjunctivitis is sustained without application to the skin during the daytime.

When the pharmaceutical composition of the present invention is used as ointment formulation, cream formulation or gel formulation, the container for the pharmaceutical composition is not particularly limited, but the pharmaceutical composition may be contained in a tube, a bottle, a can or any other container. The material of container is not particularly limited, but the container may be a resin container made of a material such as polyethylene (PE), polypropylene (PP), or polyethylene terephthalate (PET), polybuthylene terephthalate (PBT), polypropylene and polyethylene copolymer, polyvinyl chloride, acrylic resin, polystyrene, polycyclic olefin copolymer, a metal container made of a material such as aluminum, or a laminate container produced by laminating several materials such as resin, paper, aluminum foil. For example, when the material of a resin container is polyethylene, a container made of low-density polyethylene (LDPE), liner low-density polyethylene (LLDPE), medium-density polyethylene (MDPE) or high-density polyethylene (HDPE), which is polyethylene classified by its density, may be used.

The pharmaceutical composition may be used both when wearing hard contact lenses and when wearing soft contact lenses.

Hereinafter, the present invention is illustrated in Formulation Examples and Examples in order to make it easy to understand the present invention. The present invention, however, is not intended to be limited thereto by any means.

In the following Examples, condensed ricinolenic acid polyglyceryl ester, mixture of polyoxyethylene arachyl ether and stearyl alcohol, glycerol monostearate, polyglyceryl monooleate, polyethylene glycol monostearate, polyoxyethylene hydrogenated castor oil 60 and polysorbate 80 used the following substances, respectively. The present invention, however, is not intended to be limited thereto by any means.
Condensed ricinolenic acid polyglyceryl ester: NIKKOL Hexaglyn PR-15
Mixture of polyoxyethylene arachyl ether and stearyl alcohol: NIKKOL WAX230
Glycerol monostearate: Glycerol monostearate purchased from FUJIFILM Wako Chemicals Co.
Polyglyceryl monooleate: NIKKOL DGMO-CV
Polyethylene glycol monostearate: NIKKOL MYS-2V
Polyoxyethylene hydrogenated Castor Oil 60: NIKKOL HCO-60
Polysorbate 80: NIKKOL TO-10MV

### Formulation Examples

Typical examples of formulations comprising the pharmaceutical composition of the present invention are shown below. The amount of each ingredient formulated in the following formulation examples is the amount in 100 g of the composition. Also, the term "% (w/w)" refers to the amount (g) of each ingredient in 100 g of the composition.

### [Formulation Example 1]

| | |
|---|---|
| Epinastine | 1.0 g |
| Squalane | 5.0 g |
| Light Liquid Paraffin | 5.0 g |
| Ceresin | 3.0 g |
| White Petrolatum | 10.0 g |
| White Beeswax | 1.0 g |
| Glycerin Fatty Acid Ester | 5.0 g |
| Dibutylhydroxytoluene | 0.1 g |
| Disodium Edetate Hydrate | 0.5 g |
| Concentrated Glycerin | 15.0 g |
| Purified Water | q.s. |

### [Formulation Example 2]

| | |
|---|---|
| Epinastine Hydrochloride | 0.75 g |
| Cetyl Alcohol | 0.5 g |
| White Petrolatum | 8.0 g |
| Isopropyl Myristate | 4.0 g |
| Polysorbate 80 | 0.5 g |
| Dibutylhydroxytoluene | 0.2 g |
| 2-Mercaptobenzimidazole | 0.05 g |
| Propylene Glycol | 5.0 g |
| Sodium Hydroxide | q.s. |
| Purified Water | q.s. |

### [Formulation Example 3]

| | |
|---|---|
| Epinastine Hydrochloride | 0.5 g |
| Cetyl Alcohol | 2.0 g |
| Squalane | 10.0 g |
| Glycerin Fatty Acid Ester | 4.0 g |
| Polyoxyethylene Hydrogenated Castor Oil | 0.5 g |
| 2-Mercaptobenzimidazole | 0.3 g |
| Octyldodecanol | 5.0 g |
| Disodium Edetate Hydrate | 0.5 g |
| Sodium Hydroxide | q.s. |
| Purified Water | q.s. |

In addition, Formulation Examples 4 to 19 are shown in Tables 1 and 2.

**[Table 1]**

| Ingredient [%(w/w)] | Formulation Example | | | | | | | |
|---|---|---|---|---|---|---|---|---|
| | 4 | 5 | 6 | 7 | ' 8 | 9 | 10 | 11 |
| Epinastine Hydrochloride | 0.5 | 0.5 | 0.5 | 0.5 | 0.5 | 0.5 | 0.5 | 0.5 |
| White Petrolatum | 15 | 5.0 | 10 | 8.0 | 10 | 10 | 8.0 | 5.0 |
| Light Liquid Paraffin | 3.0 | 5.0 | 5.0 | 8.0 | 10 | 8.0 | 13 | 8.0 |
| Squalane | 5.0 | 5.0 | 8.0 | 8.0 | - | 8.0 | 3.0 | 8.0 |
| Mixuture of Paraffin and Microcrystalline wax | 3.5 | 4.0 | 3.5 | 2.5 | 5.0 | 3.0 | 5.0 | 3.0 |
| White Beeswax | 1.0 | 1.0 | 1.5 | 1.5 | 1.5 | 1.5 | 1.0 | 1.0 |
| Condensed Ricinolenic Acid Polyglyceryl Ester | 4.0 | 5.5 | 5.0 | 4.5 | 5.5 | 5.0 | 6.0 | 4.0 |
| 2-Mercaptobenzimidazole | 0.05 | 0.1 | - | 0.05 | 0.1 | 0.05 | 0.1 | 0.05 |
| Dibutylhydroxytoluene | 2.0 | - | 2.0 | - | 1.0 | - | - | - |
| Disodium Edetate Hydrate | 0.1 | 0.05 | 0.03 | 0.05 | 0.03 | 0.1 | 0.05 | 0.03 |
| Concentrated Glycerin | 10 | 15 | 10 | 12 | 10 | 8.0 | 15 | 13 |
| Sodium Chloride | 0.5 | 0.25 | 0.3 | 0.4 | 0.2 | 0.5 | 0.45 | 0.35 |
| Sodium Hydroxide | q.s. | | | | | | | |
| Purified Water | q.s. | | | | | | | |

**[Table 2]**

| Ingredient [%(w/w)] | Formulation Example | | | | | | | |
|---|---|---|---|---|---|---|---|---|
| | 12 | 13 | 14 | 15 | 16 | 17 | 18 | 19 |
| Epinastine Hydrochloride | 0.5 | 0.5 | 0.5 | 0.5 | 0.5 | 0.5 | 0.5 | 0.5 |
| White Petrolatum | 10 | 10 | 15 | 5.0 | 8.0 | 10 | 15 | 15 |
| Light Liquid Paraffin | 5.0 | 8.0 | - | 10 | 8.0 | 5.0 | 5.0 | - |
| Squalane | 8.0 | - | 8.0 | 5.0 | - | 5.0 | 3.0 | 5.0 |
| White Beeswax | 1.0 | 1.0 | 1.0 | - | 2.0 | 2.0 | 2.0 | 2.0 |
| Polyoxyl 40 Stearate | 5.0 | - | - | 5.0 | - | - | - | - |
| Polyoxyethylene Hydrogenated Castor Oil 60 | - | 5.0 | 5.0 | - | - | - | - | - |
| Polyoxyethylene (20) polyoxypropylene (4) cetyl Ether | 1.0 | - | 3.0 | 3.0 | - | - | - | - |
| Sorbitan Oleate | - | - | - | - | 4.0 | - | - | - |
| Glyceryl Stearate | - | - | - | - | - | 5.0 | - | - |
| Polyglyceryl Monooleate | - | - | - | - | - | - | 5.0 | 5.0 |
| 2-Mercaptobenzimidazole | 0.05 | 0.05 | 0.05 | 0.05 | 0.05 | 0.05 | 0.05 | 1.0 |
| Dibutylhydroxytoluene | 2.0 | 2.0 | 2.0 | - | 2.0 | 2.0 | 2.0 | - |
| Cetyl Alcohol | 5.0 | 8.0 | 8.0 | 8.0 | 8.0 | - | - | 5.0 |
| Disodium Edetate Hydrate | - | - | - | 0.05 | 0.05 | 0.05 | 1.0 | 1.0 |
| Concentrated Glycerin | 5.0 | - | - | - | 15 | 20 | 10 | 20 |
| Macrogol 400 | 20 | 20 | 20 | 20 | - | - | - | - |
| Sodium Chloride | - | - | - | - | 0.5 | 0.5 | 0.5 | 0.5 |
| Sodium Citrate Hydrate | 0.5 | 0.5 | 0.5 | 0.5 | - | - | - | - |
| Sodium Hydroxide | q.s. | | | | | | | |
| Purified Water | q.s. | | | | | | | |

### Examples

### 1. Drug efficacy evaluation test in allergic conjunctivitis model (1)

In order to evaluate the anti-allergic effect of the pharmaceutical composition of the present invention on allergic conjunctivitis, an allergic conjunctivitis model was produced using a guinea pig and the therapeutic effect of the pharmaceutical composition in the model was studied.

### (1) Preparation of Test Formulation

According to a commonly-used method, epinastine hydrochloride, which is an active ingredient, oil ingredients of white petrolatum, light liquid paraffin, squalane, paraffin and microcrystalline wax mixture, white beeswax and glycerin fatty acid ester, disodium edetate hydrate, 2-mercaptobenzimidazole, sodium hydroxide, sodium chloride, concentrated glycerin, and purified water were mixed to prepare test formulations 1 to 3 in the form of water-in-oil emulsion. Each amount of epinastine hydrochloride in test formulations 1 to 3 was adjusted to 0.005% (w/w), 0.05% (w/w) and 0.5% (w/w), respectively, the amount of sodium hydroxide added was appropriately adjusted depending on the amount of epinastine hydrochloride, and the remaining additives were added in equal amounts for each formulation. In addition, test formulation 4 without epinastine hydrochloride and sodium hydroxide in the form of water-in-oil emulsion was prepared according to a similar method to the method of preparing test formulations 1 to 3 except that epinastine hydrochloride and sodium hydroxide are added. Also, "ALESION^{®} LX Ophthalmic Solution 0.1%" marketed in Japan was used as test formulation 5.

### (2) Production of conjunctivitis model and evaluation test method

Guinea pigs (Male, Hartley) were randomly classified into the administration groups of the test formulations in emulsion form (the administration groups of test formulations 1 to 4) and the administration group of the test formulation in ophthalmic solution form (the administration group of test formulation 5) (each group: several guinea pigs). In the administration groups of the test formulations in emulsion form (the administration groups of test formulations 1 to 4), the guinea pigs were given general anesthesia by intramuscular injection with a mixed anesthetic solution of ketaral and seractol, and then body hair around the right eyelid of each guinea pig was shaved with clippers and an electric razor. Conjunctivitis was induced by administering histamine solution to the right eye of each guinea pig in each administration group of the test formulations to produce an allergic conjunctivitis model.

In the administration groups of test formulations in emulsion form (the administration groups of test formulations 1 to 4), each guinea pig was given general anesthesia, the test formulation was applied to the upper and lower eyelids of the right eye of the guinea pig in an amount of 15 µL each 24 hours before the ophthalmic administration of histamine solution, and then the guinea pig was fitted with an Elizabethan collar. Whereas, in the administration group of the test formulation in ophthalmic solution form (the administration group of test formulation 5), the test formulation was administered to the right eye of each guinea pig in an amount of 10 µL 8 hours before the ophthalmic administration of histamine solution. Before the ophthalmic administration of histamine solution, the guinea pigs in each administration group of the test formulations were given Evans blue solution intravenously in the ear under isoflurane inhalation anesthesia.

After the ophthalmic administration of histamine solution, the guinea pigs in each administration group of the test formulations were euthanized under isoflurane inhalation anesthesia, and the right eyes and eyelid tissues were removed therefrom. The weight of each removed tissue was measured, and then the removed tissue was immersed in a dye extraction solution (acetone solution containing sodium sulfate) to extract the dye.

The dye extraction solution in which the removed tissue is immersed was centrifuged, the supernatant was used as a sample, and then the absorbance of the sample was measured at 620 nm by a spectrophotometer. According to a commonly-used method, the mean value and standard error of the dye leakage amount per the weight of conjunctival tissue (µg/g) were calculated for each administration group of the test formulations.

### (3) Test results and discussion

The test results are shown in Table 3.

**[Table 3]**

| Administration Group | Dye Leakage Amount (µg/g) |
|---|---|
| Test formulation 1 | 10.9 ± 0.3 |
| Test formulation 2 | 7.3 ± 0.3 |
| Test formulation 3 | 3.7 ± 0.1 |
| Test formulation 4 | 13.8 ± 0.1 |
| Test formulation 5 | 6.0 ± 0.1 |

Test formulations 1 to 3 applied to the eyelid skin had lower dye leakage amounts than test formulation 4 without epinastne or a salt thereof which is an active ingredient. Hence, it was showed that test formulations 1 to 3 produced the therapeutic effect on allergic conjunctivitis.

Also, the therapeutic effect at the time of 8 hours after the ophthalmic administration of test formulation 5 was equal or greater than the therapeutic effects at the time of 24 hours after the ophthalmic administration of test formulations 2 to 3. That is, it was shown that the therapeutic effects of test formulations 2 and 3 were kept longer than that of test formulation 5.

Accordingly, in light of the fact that the administration frequency of test formulation 5, which is actually used as an agent for treating allergic conjunctivitis, is twice daily, the results suggest that the pharmaceutical composition of the present invention is sufficiently effective as an agent for treating allergic conjunctivitis when applied to the skin once daily.

### 2. Drug efficacy evaluation test in allergic conjunctivitis model (2)

According to a similar procedure to the above "Drug efficacy evaluation test in allergic conjunctivitis model (1)", the therapeutic effect of the pharmaceutical composition of the present invention was studied.

### (1) Preparation of test formulation

According to a commonly-used method, epinastine hydrochloride, which is an active ingredient, each additive ingredient and purified water were mixed at the concentrations in Table 4 below to prepare test formulations 6 to 13. Test formulations 6, 7, 10 and 11 are in the form of water-in-oil emulsion, and test formulations 8, 9, 12 and 13 are in the form of oil-in-water emulsion.

**[Table 4]**

| Ingredient [%(w/w)] | Test Formulation | | | | | | | |
|---|---|---|---|---|---|---|---|---|
| | 6 | 7 | 8 | 9 | 10 | 11 | 12 | 13 |
| Epinastine Hydrochloride | 0.5 | 0.5 | 0.5 | 0.5 | 0.5 | 0.5 | 0.5 | 0.5 |
| White Petrolatum | 3.0 | 5.0 | 3.0 | 3.0 | - | 5.0 | - | - |
| Light Liquid Paraffin | - | 8.0 | - | 5.0 | 13 | 8.0 | - | - |
| Squalane | - | - | 5.0 | - | - | 8.0 | - | - |
| Mixture of Paraffin and Microcrystalline Wax | - | - | - | - | - | 3.0 | - | - |
| White Beeswax | - | - | - | - | - | 30 | - | - |
| Condensed Ricinolenic Acid Polyglyceryl Ester | 5.0 | 4.0 | - | - | - | - | - | - |
| Medium-Chain Triglyceride | 5.0 | | - | - | | - | 20 | - |
| Mixture of Polyoxyethylene Arachyl Ether and Stearyl Alcohol | - | - | 5.0 | | - | - | - | - |
| Glycerol Monostearate | - | - | - | 5.0 | - | - | - | - |
| Polyglyceryl Monooleate | - | - | - | - | - | 4 | | - |
| Polyethylene Glycol Monostearate | - | - | - | - | 5 | - | - | - |
| Diethyl Sebacate | - | - | - | - | - | - | - | 20 |
| Polyoxyethylene Hydrogenated Castor Oil 60 | - | - | - | - | - | - | 5.0 | - |
| Polysorbate 80 | - | - | - | - | - | - | - | 5.0 |
| Carbopol | - | - | - | - | - | - | 2.0 | - |
| Hydroxyethyl Cellulose | - | - | - | - | - | - | - | 5.0 |
| Concentrated Glycerin | 15 | 13 | 15 | 15 | - | 13 | - | - |
| 1,3-Butylene Glycol | - | - | - | - | 15 | - | 10 | 10 |
| Sodium Hydroxide | q. s. | | | | | | | |
| Purified Water | q.s. | | | | | | | |

### (2) Production of conjunctivitis model and evaluation test method

According to a similar method to that of the above "Drug efficacy evaluation test in allergic conjunctivitis model (1)", an allergic conjunctivitis model was produced (each test preparation: several models), and the mean value and standard error of the dye leakage amount per the weight of conjunctival tissue (µg/g) were calculated for test formulations 6 to 13.

### (3) Test results and discussion

The test results are shown in Table 5.

**[Table 5]**

| Administration Group | Dye Leakage Amount (µg/g) |
|---|---|
| Test formulation 6 | 3.9 ± 0.2 |
| Test formulation 7 | 3.3 ± 0.3 |
| Test formulation 8 | 3.9 ± 0.2 |
| Test formulation 9 | 5.0 ± 0.3 |
| Test formulation 10 | 3.7 ± 0.2 |
| Test formulation 11 | 5.1 ± 0.4 |
| Test formulation 12 | 7.6 ± 0.4 |
| Test formulation 13 | 5.2 ± 0.2 |

When compared with the dye leakage amount (µg/g) for test formulation 4 (the formulation without an active ingredient, epinastine or a salt thereof), it was shown that test formulations 6 to 13 applied to the eyelid skin had lower dye leakage amounts, although they were different in additive ingredients from test formulation 4. Hence, the results suggest that the pharmaceutical composition of the present invention is sufficiently effective as an agent for treating allergic conjunctivitis when applied to the skin once daily.

### 3. Skin imitation evaluation test

The skin imitation when the composition comprising epinastine or a salt thereof as an active ingredient is applied to the eyelid skin was studied using a rabbit.

### (1) Preparation of Test Formulation

As the test formulations, test formulations 3 and 4 as well as test formulation 14 in the form of water-in-oil emulsion prepared in a similar method to that of the above "Drug efficacy evaluation test in allergic conjunctivitis model (1)" were used. Test formulations 3 and 4 were prepared again in a similar method to that of the above "Drug efficacy evaluation test in allergic conjunctivitis model (1)", and test formulation 14 was prepared by adjusting the amount of epinastine hydrochloride to 1% (w/w), adjusting the amount of sodium hydroxide added depending on the amount of epinastine hydrochloride and adding the remaining additives in equal amounts to test formulations 3 and 4.

### (2) Evaluation test method

The rabbits acclimatized at appropriate (Male, Kbl:JW) were given general anesthesia by intramuscular injection with a mixed anesthetic solution of ketaral and seractol, and then the hair in the area of the upper eyelid about 5 mm away from the edge of the rabbit's eyelid was shaved with clippers, an electric shaver or an eyebrow shaver. In addition, the eyelashes and any hairs that may touch the administration site were removed. The rabbits whose hairs were removed were fitted with Elizabethan collars and were classified into the non-treatment group and each administration group of test formulations 3, 4 and 14. In each administration group, the test formulation (30 µL) was applied to the upper eyelid of each rabbit using a microman. The condition of each rabbit was observed periodically, and the skin reactivity at the applied site of the test formulation was determined at about 3, 6 and 24 hours after administration according to the criteria for determining the skin reactivity by the Draize test.

The criteria for determining the skin reactivity by the Draize test is scored based on the following symptoms.

### <Erythema eschar formation (Erythema score)>

0: No erythema
1: Very slight erythema (barely perceptible)
2: Well-defined erythema
3: Moderate-to-severe erythema
4: Severe erythema (beet redness) to slight eschar formation (injuries in depth)

### <Edema formation (Edema score)>

0: No edema
1: Very slight edema (barely perceptible)
2: Well-defined edema (edges of area well-defined by definite raising)
3: Moderate edema (raised approximately 1 mm)
4: Severe edema (raised >1 mm and extending beyond area of exposure)

### (3) Test results and discussion

The erythema score and edema score at each time point (3, 6 and 24 hours after administration) for the non-treatment group (n = 2) and the administration group of the test formulation 4 (n = 4) were 0. That is, no clear indication of skin irritation was observed. Hence, it was shown that the base of the formulation in the form of water-in-oil emulsion used in this test was of very low safety concerns.

In the administration group of test formulation 3 (n = 5), the edema score at each time point (3, 6 and 24 hours after administration) was 0 and the erythema scores at 3 and 6 hours after administration were 0, but the erythema score in one eyelid at 24 hours after administration was 1. In the administration group of test formulation 14 (n = 5), the edema score at each time point (3, 6 and 24 hours after administration) was 0, but the erythema scores in all of 5 eyelids at 3, 6 and 24 hours after administration were 1.

Hence, it was shown that when a composition comprising epinastine or a salt thereof as an active ingredient is applied to the eyelid skin, higher concentrations of epinastine or a salt thereof tended to cause skin irritation, that the concentration of epinastine or a salt thereof was preferably less than 1% (w/w) in view of the safety, and that the concentration of epinastine or a salt thereof was particularly preferably 0.5% (w/w). The skin irritation observed by the administration of test formulations 3 and 14 were both mild, and thus it was thought that both formulations were of low clinical safety concerns.

### 4. Drug efficacy evaluation test in human

In order to evaluate the anti-allergic effect of the pharmaceutical composition of the present invention on allergic conjunctivitis in human, a clinical test was performed and the therapeutic effect of the pharmaceutical composition in human was studied.

### (1) Preparation of test formulation

According to a commonly-used method, epinastine hydrochloride, which is an active ingredient, oil ingredients of white petrolatum, light liquid paraffin, squalane, paraffin and microcrystalline wax mixture, white beeswax and glycerin fatty acid ester, disodium edetate hydrate, 2-mercaptobenzimidazole, sodium hydroxide, sodium chloride, concentrated glycerin, and purified water were mixed to prepare cream formulations 1 and 2. The amounts of epinastine hydrochloride in the cream formulations 1 and 2 were adjusted to 0.05% (w/w) and 0.5% (w/w), respectively, the amount of sodium hydroxide added in each cream formulation was appropriately adjusted depending on the amount of epinastine hydrochloride, and the remaining additives were added in equal amounts for each formulation. In addition, cream formulation 3 for placebo without epinastine hydrochloride and sodium hydroxide was prepared according to a similar method to the method of cream formulations 1 and 2 except that epinastine hydrochloride and sodium hydroxide are added.

### (2) Test method

The healthy adult volunteers with allergic reactivity induced by cedar pollen antigens were recruited as subjects (8 volunteers per cohort). The optimal antigen concentration for each subject was determined in advance, and then each cream formulation (Cohort 1: cream formulation 1, Cohort 2: cream formulation 2) or cream formulation 3 for placebo was randomly assigned to the left or right eyelid. In the double blind test, cream formulation 1 or 2 was applied to one eyelid (upper and lower eyelids) and cream formulation 3 for placebo was applied to the other eyelid (upper and lower eyelids), and the antigen induction was performed at the time of 25 hours after the cream formulation was applied (corresponding to the administration interval when administrated once daily).

### (3) Method of evaluating allergic symptoms caused by antigen induction

The ocular itching and conjunctival hyperemia (ocular conjunctival hyperemia and eyelid conjunctival hyperemia) were evaluated by scoring them according to the criteria based on the severity of symptoms. The score for ocular itching is expressed by a 5-point scale from 0 to 4 and the score for conjunctival hyperemia is expressed by a 7-point scale from 0 to 6 (the total score of ocular conjunctival hyperemia (0 to 3) and eyelid conjunctival hyperemia (0 to 3)).

### (4) Test results and discussion

The average ocular itching score at 3 points (3, 5 and 10 minutes) after the antigen induction and the average conjunctival hyperemia score at 3 points (5, 10 and 20 minutes) after the antigen induction are shown in Table 6. The average values and standard deviations in the table are calculated according to a commonly-used statistical processing.

**[Table 6]**

| Cohort | Administration Group | Average value (Standard deviation) | |
|---|---|---|---|
| | | *Ocular* Itching score | Conjunctival hyperemia score |
| 1 | Cream Formulation 1 | 0.63 (0.653) | 2.50 (1.808) |
| | Cream Formulation 3 (Placebo) | 1.17 (0.873) | 3.00 (0.617) |
| 2 | Cream Formulation 2 | 0.71 (0.375) | 1.67 (1.425) |
| | Cream Formulation 3 (Placebo) | 1.92 (0.154) | 3.00 (0.713) |

As shown in Table 6, cream formulations 1 and 2 were observed to show differences from cream formulation 3 (placebo) in ocular itching score and conjunctival hyperemia score at a dose equivalent to once daily. In particularly, cream formulation 2 comprising 0.5% (w/w) of epinastine hydrochloride showed significantly greater differences in both scores.

Hence, it was shown that the pharmaceutical composition of the present invention was effective as an agent for treating allergic conjunctivitis in human. In addition, in this test, none of test formulations caused any side effects when applied to the eyelid and the test formulations were well tolerated as pharmaceutical products.

Allergic conjunctivitis is a disease that causes the inflammation of conjunctiva, that is, the mucous membrane covering the back of the eyelid and the white part of eyes due to an allergen such as pollen. The current treatment of allergic conjunctivitis is based on direct administration of an eye drop to the surface of eyes. On the other hand, the eyelid skin is an organ that covers and protects the eyeballs from above and below continuously from the skin of face. As shown in the above test results, the cream formulation comprising lower concentration of epinastine or a salt thereof produced the therapeutic effect useful as an agent for treating allergic conjunctivitis even when the cream formulation was applied to the eyelid skin once daily and the safety concerns could be minimized even when the cream formulation was applied to the thin and irritation-sensitive eyelid skin. They were surprising results.

### 5. Formulation property evaluation test

The usability of formulation when the pharmaceutical composition of the present invention is used as an ophthalmic formulation for application to the skin was focused to evaluate the formulation property of the pharmaceutical composition of the present invention.

### (1) Preparation of test formulation

As the test formulations, test formulations 6 to 13 of the above "2. Drug efficacy evaluation test in allergic conjunctivitis model (2)" were used. In addition, according to a commonly-used method, epinastine hydrochloride, which is an active ingredient, each additive ingredient, and purified water were mixed at the concentrations in Tables 7 to 9 below to prepare test formulations 15 to 20 in the form of water-in-oil emulsion and test formulations 21 to 29 in the form of oil-in-water emulsion.

**[Table 7]**

| Ingredient [%(w/w)] | Test Formulation | | | | | |
|---|---|---|---|---|---|---|
| | 15 | 16 | 17 | 18 | 19 | 20 |
| Epinastine Hydrochloride | 0.5 | 0.5 | 0.5 | 0.5 | 0.5 | 0.5 |
| White Petrolatum | - | - | - | 5.0 | 5.0 | 5.0 |
| Light Liquid Paraffin | 13 | 13 | 13 | 8.0 | 8.0 | 8.0 |
| Squalane | - | - | - | 8.0 | 8.0 | 8.0 |
| Paraffin and Microcrystalline Wax Mixture | 10 | 20 | 30 | 3.0 | 3.0 | 3.0 |
| White Beeswax | - | - | - | 5.0 | 10 | 20 |
| Polyethylene Glycol Monostearate | 5.0 | 5.0 | 5.0 | - | - | - |
| Polyglyceryl Monooleate | - | - | - | 4.0 | 4.0 | 4.0 |
| Concentrated Glycerin | - | - | - | 13 | 13 | 13 |
| 1,3-Butylene Glycol | 15 | 15 | 15 | - | - | - |
| Sodium Hydroxide | q.s. | | | | | |
| Purified Water | q.s. | | | | | |

**[Table 8]**

| Ingredient [%(w/w)] | Test Formulation | | | | | |
|---|---|---|---|---|---|---|
| | 21 | 22 | 23 | 24 | 25 | ' 26 |
| Epinastine Hydrochloride | 0.5 | 0.5 | 0.5 | 0.5 | 0.5 | 0.5 |
| White Petrolatum | 3.0 | 3.0 | 3.0 | 3.0 | 3.0 | 3.0 |
| Light Liquid Paraffin | 5.0 | 5.0 | 5.0 | - | - | - |
| Squalane | - | - | - | 5.0 | 5.0 | 5.0 |
| Mixuture of Paraffin and Microcrystalline Wax | - | 25 | - | - | - | - |
| White Beeswax | - | - | 25 | - | - | - |
| Polyoxyethylene Hydrogenated Castor Oil 60 | 5.0 | 5.0 | 5.0 | - | - | - |
| Mixture of Polyoxyethylene Arachyl Ether and Stearyl Alcohol | - | - | - | 5.0 | 5.0 | 5.0 |
| Cetyl Alcohol | - | - | - | 10 | 20 | 30 |
| Hydroxyethyl Cellulose | 10 | 5.0 | 10 | - | - | - |
| Concentrated Glycerin | 10 | 10 | 10 | 15 | 15 | 15 |
| Sodium Hydroxide | q.s. | | | | | |
| Purified Water | q.s. | | | | | |

**[Table 9]**

| Ingredient [%(w/w)] | Test Formulation | | |
|---|---|---|---|
| | 27 | 28 | 29 |
| Epinastine Hydrochloride | 0.5 | 0.5 | 0.5 |
| White Petrolatum | 3.0 | - | - |
| Light Liquid Paraffin | - | 13 | - |
| Squalane | 5.0 | - | - |
| Medium-Chain Triglyceride | - | - | 20 |
| Polyoxyethylene Hydrogenated Castor Oil 60 | 4.0 | 4.0 | 4.0 |
| Concentrated Glycerin | 13 | 13 | 15 |
| Sodium Hydroxide | q.s. | | |
| Purified Water | q.s. | | |

### (2) Evaluation test method

The eyelid skin is a very thin and sort tissue. When the eyelid skin is strongly pressed, it may cause pain to the eyeball located directly under the eyelid skin and affect its vision. Thus, assuming that the pharmaceutical composition of the present invention is applied to the eyelid skin, the usability of formulation when each test formulation was applied to artificial skin was evaluated for 4 subjects. In addition, the viscosity of each test formulation was measured.

Subjects took an appropriate amount (approximately the size of a grain of rice) of each test formulation contained in ointment jars with their index finger and performed the operation for applying to the skin by extending the formulation to the left and right over an area of about 2 cm x 2 cm on the artificial skin. As for the usability of formulation when applied to the skin, the strength of pressure and the stretchability of formulation were each scored by each of the 3-point scales below, and the total of the scores was used as the evaluation score for each test formulation. The maximum evaluation score is 16 (maximum score 4 per subject x 4 subjects). The maximum evaluation scores for the test formulations 27 to 29 were 8 (maximum score 4 per subject x 2 subjects).

### <Strength of Pressure>

2: Formulation can be applied to the skin without pressure.
1: Formulation can be applied to the skin with slight pressure.
0: Formulation can be applied to the skin with strong pressure/Formulation cannot be applied to the skin even with strong pressure.

### <Stretchability of Formulation>

2: Formulation can be easily and uniformly applied to the skin.
1: Formulation can be uniformly applied to the skin.
0: Formulation cannot be uniformly applied to the skin.

The artificial skin used in this test used commercially available Spacerail L988 that is a substance felt to most closely resemble the flexibility of eyelid skin.

According to the viscosity measurement method described in the General Test Method of the Japanese Pharmacopoeia, 17 Edition, the viscosity of each test formulation was measured using a Cone-Plate Rotational Viscometer (Cone-Plate Viscometer) at a temperature of 20 ± 0.1 degrees Celsius.

### (3) Test results and discussion

The test results are shown in Table 10 (sorted by viscosity).

**[Table 10]**

| Test Formulation | Evaluation score | Viscosity (Pa·s) |
|---|---|---|
| 27 | 8/8 | 0.0033 |
| 28 | 8/8 | 0.0035 |
| 29 | 8/8 | 0.0040 |
| 9 | 16/16 | 0.30 |
| 10 | 16/16 | 0.64 |
| 12 | 16/16 | 0.67 |
| 8 | 16/16 | 0.96 |
| 26 | 16/16 | 3.8 |
| 13 | 16/16 | 10.6 |
| 24 | 16/16 | 11.0 |
| 15 | 16/16 | 16.4 |
| 25 | 12/16 | 17.7 |
| 16 | 12/16 | 32.5 |
| 6 | 16/16 | 42.9 |
| 19 | 14/16 | 62.4 |
| 17 | 11/16 | 83.9 |
| 18 | 13/16 | 93.9 |
| 21 | 14/16 | 103 |
| 7 | 14/16 | 132 |
| 11 | 7/16 | 152 |
| 20 | 11/16 | 155 |
| 23 | 2/16 | 160 |
| 22 | 6/16 | 255 |

As shown in Table 10, it was suggested that the usability of formulation when applied to the artificial skin was good when the viscosity of the test formulation was low, whereas the usability of formulation when applied to the artificial skin tended to worsen as the viscosity of the test formulation increased. The eyelid skin was very thin and soft compared to the skin in any other areas, and thus it was difficult to apply a formulation to the eyelid skin. Also, the eyeball was located directly under the eyelid skin tissue, and thus the formulation could not be applied to the eyelid skin while adding strong pressure. Hence, when the pharmaceutical composition of the present invention is used as an ophthalmic formulation for application to the skin, it is thought that the formulation property so that a formulation can be easily and uniformly applied to the eyelid skin without the irritation to eyeball is preferably a low viscosity and more preferably a viscosity of, for example, 150 Pa·s or less.

### INDUSTRIAL APPLICABILITY

The present invention provides a pharmaceutical composition for application to the skin comprising epinastine or a salt thereof that produces sufficient therapeutic effects even at low concentrations and reduced administration frequency, minimizes safety concerns and has good usability of formulation.

## Claims

1. A pharmaceutical composition for application to the skin comprising epinastine or a salt thereof in a concentration of 0.05 to 1% (w/w) as an active ingredient.

2. The pharmaceutical composition according to claim 1 for use in the administration to eyelid skin.

3. The pharmaceutical composition according to claim 1 or 2 for use in the administration to a patient once daily.

4. The pharmaceutical composition according to any one of claims 1 to 3 for treating allergic conjunctivitis.

5. The pharmaceutical composition according to any one of claims 1 to 4 with a viscosity of 150 Pa · s or less at 20 degrees Celsius.

6. The pharmaceutical composition according to any one of claims 1 to 5, wherein the pharmaceutical composition is ointment formulation, cream formulation or gel formulation.

7. The pharmaceutical composition according to any one of claims 1 to 5, wherein the pharmaceutical composition is cream formulation.

8. The pharmaceutical composition according to any one of claims 1 to 7, wherein the pharmaceutical composition is water-in-oil emulsion.

9. The pharmaceutical composition according to any one of claims 1 to 8, wherein the concentration of the epinastine or a salt thereof is 0.05% (w/w).

10. The pharmaceutical composition according to any one of claims 1 to 8, wherein the concentration of the epinastine or a salt thereof is 0.5% (w/w).

11. The pharmaceutical composition according to any one of claims 1 to 10, wherein the epinastine or a salt thereof is epinastine hydrochloride.

12. The pharmaceutical composition according to any one of claims 1 to 10, wherein the epinastine or a salt thereof is epinastine.

13. The pharmaceutical composition according to any one of claims 1 to 12 which comprises one or more oil ingredients selected from the group consisting of a hydrocarbon, a wax, an oil and fat, an aliphatic carboxylic acid or a salt thereof, a fatty acid ester and a higher alcohol.

14. The pharmaceutical composition according to any one of claims 1 to 12 which comprises a surfactant.

15. The pharmaceutical composition according to claim 14, wherein the surfactant is glycerin fatty acid ester.

16. The pharmaceutical composition according to claim 14 or 15, wherein the surfactant has a HLB of 3.0 to 6.0.

17. The pharmaceutical composition according to any one of claims 1 to 16, wherein the pharmaceutical composition is free of a paraben.

18. A pharmaceutical composition for application to the skin comprising epinastine or a salt thereof in a concentration of 0.05 to 0.5% (w/w) as an active ingredient wherein the pharmaceutical composition is cream formulation in the form of water-in-oil emulsion for use in the administration to the eyelid skin once daily.

19. A pharmaceutical composition for application to the skin comprising epinastine or a salt thereof in a concentration of 0.5% (w/w) as an active ingredient, wherein the pharmaceutical composition is cream formulation in the form of water-in-oil emulsion for use in the administration to the eyelid skin once daily.

20. A method of transferring a therapeutically effective amount of epinastine to an ocular tissue, by administering a pharmaceutical composition comprising epinastine or a salt thereof in a concentration of 0.05 to 1% (w/w) as an active ingredient to the eyelid skin.

21. A method of producing the sustained release of a therapeutically effective amount of epinastine from the eyelid skin to an ocular tissue, by administering a pharmaceutical composition comprising epinastine or a salt thereof in a concentration of 0.05 to 1% (w/w) as an active ingredient to the eyelid skin.

22. The method according to claim 20 or 21, wherein the pharmaceutical composition is administered to a patient once daily.

23. The method according to any one of claims 20 to 22, wherein the ocular tissue is conjunctiva.
